(19) Europäisches Patentamt
European Patent Office
Office européen des brevets

(11) Veröffentlichungsnummer: **0 010 242**
**B1**

(12) # EUROPÄISCHE PATENTSCHRIFT

(45) Veröffentlichungstag der Patentschrift: **29.09.82**

(21) Anmeldenummer: **79103831.8**

(22) Anmeldetag: **08.10.79**

(51) Int. Cl.³: **C 07 D 235/32,** C 07 D 403/12, C 07 D 401/12, C 07 D 413/12, A 61 K 31/415

(54) **2-Benzimidazolylcarbamidsäureester, diese enthaltende Arzneimittel sowie Verfahren zur ihrer Herstellung.**

(30) Priorität: **19.10.78 DE 2845537**

(43) Veröffentlichungstag der Anmeldung: **30.04.80 Patentblatt 80/9**

(45) Bekanntmachung des Hinweises auf die Patenterteilung: **29.09.82 Patentblatt 82/39**

(84) Benannte Vertragsstaaten: **BE CH DE FR GB IT NL SE**

(56) Entgegenhaltungen:
**DE-A-2 711 945**
**FR-A-2 234 000**
**FR-A-2 260 333**
**US-A-3 682 952**
**US-A-4 026 936**

(73) Patentinhaber: **BAYER AG, Zentralbereich Patente, Marken und Lizenzen, D-5090 Leverkusen 1, Bayerwerk (DE)**

(72) Erfinder: **Wollweber, Hartmund, Dr., In den Birken 73, D-5600 Wuppertal 1 (DE)**
Erfinder: **Kölling, Heinrich, Dr., Adlerstrasse 15, D-5657 Haan (DE)**
Erfinder: **Thomas, Herbert, Dr., Bergerheide 82 b, D-5600 Wuppertal 1 (DE)**
Erfinder: **Andrews, Peter, Dr., Am Eckbusch 35, D-5600 Wuppertal 1 (DE)**

**EP 0 010 242 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# 2-Benzimidazolylcarbamidsäureester, diese enthaltende Arzneimittel sowie Verfahren zu ihrer Herstellung.

Die vorliegende Erfindung betrifft neue 2-Benzimidazolylcarbamidsäureester, Verfahren zu ihrer Herstellung und diese Verbindungen enthaltende Arzneimittel, insbesondere Anthelmintika.

Es ist bereits bekanntgeworden, dass Benzimidazolylcarbamidsäureester, die in 5(6)-Stellung entweder unsubstituiert oder mit andern als in der vorliegenden Anmeldung beschriebenen Substituenten substituiert sind, eine anthelmintische Wirkung besitzen (siehe dazu DE-OS 2 029 637, französische Patentschrift 1 556 824, DE-OS 2 164 690, P. Actor et al., Nature 215, 321 (1967) sowie die französische Patentveröffentlichung 2 260 333). So stellen die Benzimidazolylcarbamidsäureester Parbendazol (A) und Mebendazol (B) Handelsprodukte für die gleiche Indikation dar.

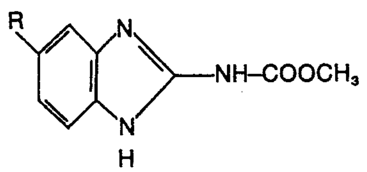

R = $C_4H_9$ = Parbendazol (A)
R = $COC_6H_5$ = Mebendazol (B)

Diese Verbindungen zeigen jedoch den Nachteil, dass sie in Wasser und anderen physiologisch verträglichen Lösungsmitteln schwer löslich sind und sich daher nicht parenteral in der Praxis anwenden lassen. Es können von ihnen zwar ölige Lösungen oder Suspensionen hergestellt werden, jedoch sind diese z.B. bei parenteraler Applikation an Grosstieren nicht ausreichend wirksam und/oder lokal unverträglich.

Die parenterale Anwendung von Anthelmintika ist jedoch für die Behandlung zahlreicher Tierspezies, besonders von Grosstieren wie Rindern, und in der humanmedizinischen Anwendung von grosser wirtschaftlicher Bedeutung.

Durch vorliegende Erfindung werden neue 2-Benzimidazolylcarbamidester der Formel

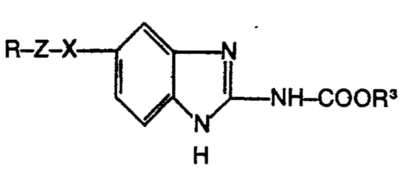

(I)

bereitgestellt,
in welcher

X für ein Schwefelatom, ein Sauerstoffatom, die SO- oder die $SO_2$-Gruppe steht,

Z für eine Alkylengruppe mit 1 bis 6 Kohlenstoffatomen steht.

R für die $NH_2$, $NHR^1$ oder die $NR^1R^2$-Gruppe steht, wobei die Substituenten $R^1$ und $R^2$ gleich oder verschieden sein können und für gegebenenfalls durch Halogen, Cyan, $C_1$–$C_4$-Alkoxy oder durch die Nitrogruppe substituiertes $C_1$–$C_6$-Alkyl, gegebenenfalls durch $C_1$–$C_4$-Alkyl, $C_1$–$C_4$-Alkoxy, Halogen, Trifluormethyl, Nitro oder Cyano substituiertes Phenyl oder gegebenenfalls durch $C_1$–$C_4$-Alkyl, $C_1$–$C_4$-Alkoxy, Halogen, Trifluormethyl, Nitro, Cyano substituiertes Phenylalkyl, wobei der Alkylteil des Phenylalkylrestes 1 bis 4 Kohlenstoffatome besitzt, stehen, oder wobei $R^1$ der Gruppen $NHR^1$ oder $NR^1R^2$ mit einem Kohlenstoffatom des Substituenten Z zu einem 5- bis 7-gliedrigen gegebenenfalls durch $C_1$–$C_4$-Alkyl substituierten Ring verbunden sein kann, oder wobei die Reste $R^1$ und $R^2$ zusammen mit dem Stickstoffatom einen gegebenenfalls durch $D_1$–$C_4$-Alkyl substituierten Azabicycloalkylrest mit 6 bis 10 Ringgliedern bilden können oder wobei R für eine gegebenenfalls durch O, S, NH oder $NR^1$ unterbrochene und/oder durch eine oder zwei Alkyl-Gruppen substituierte Cycloalkylenimino-, Cycloalkenylenimino- oder Cycloalkadienyleniminogruppe mit insgesamt 4 bis 7 Ringgliedern steht, und

$R^3$ für ($C_1$–$C_4$)-Alkyl oder für ($C_2$–$C_6$)-Alkenyl steht.

Ausserdem werden die entsprechenden physiologisch verträglichen Säureadditionssalze bereitgestellt. Die erfindungsgemässen Verbindungen und ihre Salze weisen eine ausgezeichnete anhelmintische Wirksamkeit, insbesondere auch nach oraler und nach parentaler Applikation auf.

Besonders bevorzugt sind Verbindungen der Formel (I), in welcher

X für Sauerstoff oder Schwefel steht,

Z für eine geradkettige oder verzweigte Alkylengruppe mit 1 bis 6 Kohlenstoffatomen steht,

R für die $NHR^1$ oder die $NR^1R^2$-Gruppe steht, wobei $R^1$ und $R^2$ gleich oder verschieden sein können und für Alkyl ($C_1$–$C_6$) stehen, oder wobei $R^1$ der Gruppen $NHR^1$ oder $NR^1R^2$ mit einem Kohlenstoffatom der Alkylengruppe Z zu einem 5- oder 6-gliedrigen Ring verbunden ist und $R^2$ der Gruppe $NR^1R^2$ für Alkyl ($C_1$–$C_6$) steht, oder wobei die Substituenten $R^1$ und $R^2$ zusammen mit dem durch sie eingeschlossenen Stickstoffatom einen Azabicycloalkylrest mit 6 bis 10 Ringgliedern bilden, oder wobei R für eine gegebenenfalls durch Sauerstoff, Schwefel, eine NH- oder eine $NR^4$-Gruppe unterbrochene und gegebenenfalls durch ein oder zwei Alkyl($C_1$–$C_4$)-Gruppen substituierte Cycloalkyleniminogruppe mit insgesamt 5 bis 7 Ringgliedern steht, und $R^4$ ($C_1$–$C_4$)-Alkyl bedeutet und

$R^3$ für Alkyl ($C_1$–$C_4$) steht und deren physiologisch verträglichen Salze.

Die neuen 2-Benzimidazolylcarbamidsäureester der Formel I erhält man, wenn man

a) o-Phenylen-diaminderivate der Formel

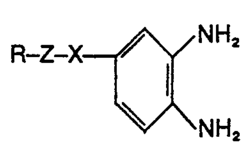

(II)

in der

R, X und Z die oben angegebene Bedeutung besitzen, mit Säurederivaten der Formel

Y–COOR³ (III)

in welcher

R³ die oben angegebene Bedeutung besitzt und

Y für einen der folgenden Reste steht:

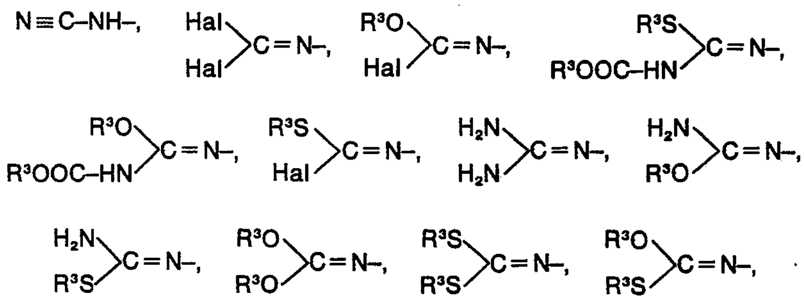

wobei R³ die oben angegebene Bedeutung besitzt und Hal für Halogen steht,
umsetzt, oder dass man

b) 2-Aminobenzimidazol-Derivate der Formel

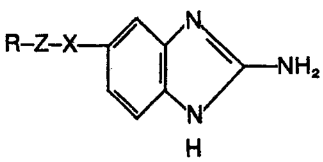

(IV)

in welcher

R, X und Z die oben angegebene Bedeutung besitzen, mit einem Kohlensäurederivat der Formel

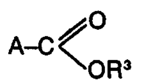

(V)

in welcher

R³ die oben angegebene Bedeutung besitzt und

A für Halogen, gegebenenfalls substituiertes Alkoxy oder den Rest OCOOR³ steht, wobei R³ wiederum die oben angegebene Bedeutung besitzt,
gegebenenfalls in Gegenwart von Säurebindemitteln umsetzt,
oder dass man zum Erhalt von Verbindungen der Formel I, bei welchen X für die SO- oder die $SO_2$-Gruppe steht,

c) Arylthiobenzimidazolylcarbamidsäureester der Formel

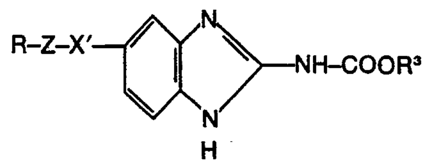

(VI)

in welcher

R und Z die oben angegebene Bedeutung besitzen und

X′ für ein Schwefelatom steht,
mit einem geeigneten Oxidationsmittel umsetzt.

Überraschenderweise zeigen die erfindungsgemässen Benzimidazolylcarbamidsäureester der Formel (I) eine ausgeprägte anthelmintische Wirkung, auch wenn man sie in Form ihrer wasserlöslichen Salze in wässriger Lösung appliziert. Dieses ist nicht möglich mit den aus dem Stand der Technik bekannten Handelsprodukten Parbendazole und Mebendazole. Die erfindungsgemässen Stoffe stellen somit eine Bereicherung der Pharmazie dar.

Die erfindungsgemässen Verbindungen der Formel (I) können in tautomeren Formen vorliegen. Siehe dazu das folgende allgemeine Schema:

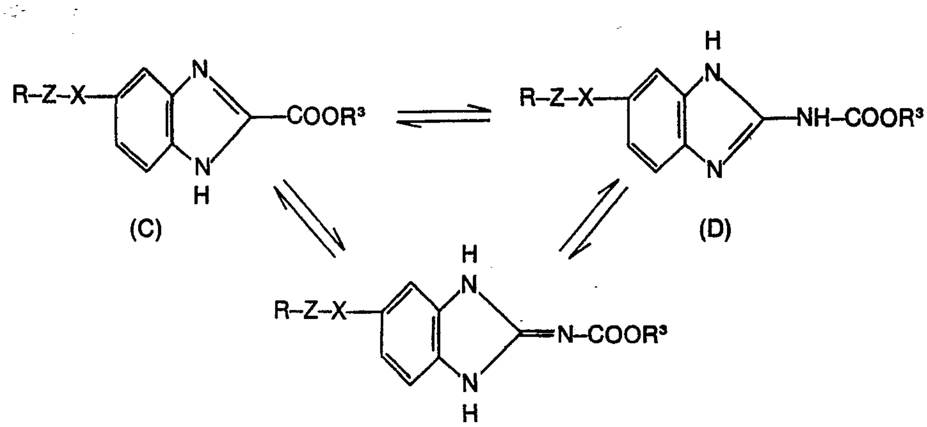

(C) (D) (E)

Die Substituentengruppierung R–Z–X ist in diesen tautomeren Formeln entweder in 5-Stellung (Formeln C und E) oder in 6-Stellung (Formel D) mit dem Benzimidazolring verbunden.

Im Anmeldungstext werden die jeweiligen Strukturformeln aus Gründen der Einheitlichkeit in allen Fällen gleichartig, nämlich entsprechend der Formel C, formuliert. Daher wird auch nomenklatorisch die Substituentengruppierung R–Z–X in dieser Anmeldung mit der 5-Stellung bezeichnet.

Verwendet man bei der Verfahrensvariante a) 4-Diäthylaminoäthylthio-1,2-phenylendiamin und N-[(Bis-chlor)-methylen]-carbamidsäuremethylester als Ausgangsstoffe, so kann der Reaktionsablauf durch das folgende Formelschema wiedergegeben werden:

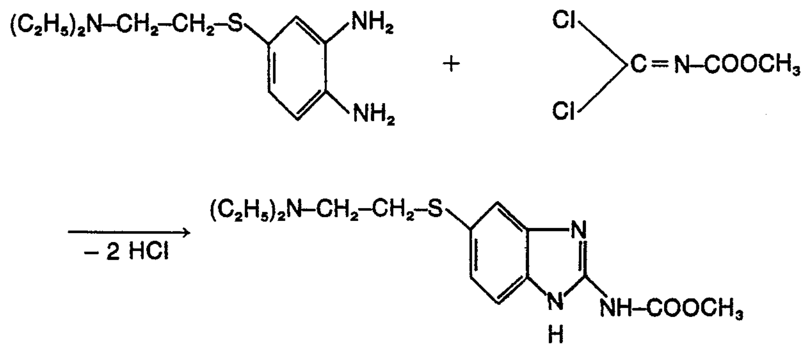


Verwendet man bei der Verfahrensvariante b) 2-Amino-5-(2-diäthylaminoäthylthio)-benzimidazol und Chlorameisensäuremethylester als Ausgangsstoffe, so kann der Reaktionsablauf durch das folgende Formelschema wiedergegeben werden:

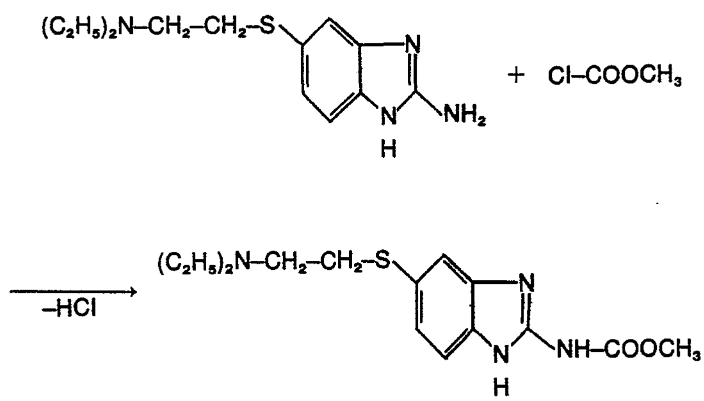


Verwendet man bei der Verfahrensvariante c) 5-(2-Diäthylaminoäthylthio)-benzimidazol-2-carbamidsäuremethylester und 1 Äquivalent Wasserstoffperoxid als Ausgangsstoffe, so kann der Reaktionsablauf durch das folgende Formelschema wiedergegeben werden:

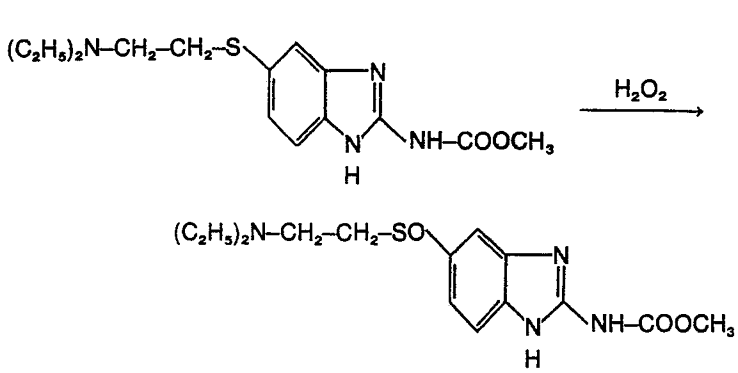

Verwendet man bei der Verfahrensvariante c) 2 Äquivalente Wasserstoffperoxid, so entsteht die entsprechende Sulfonylverbindung:

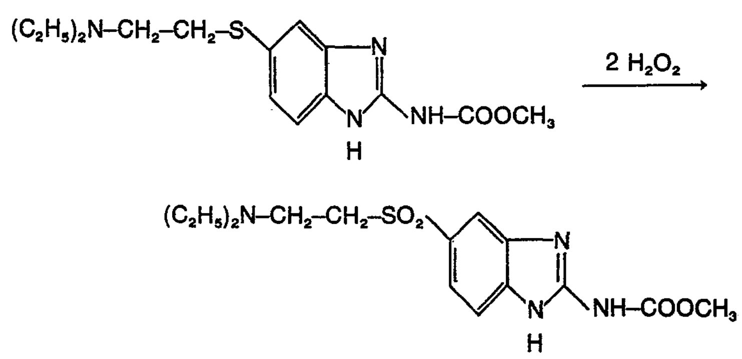


Als Alkylengruppen ($C_1$–$C_6$) seien folgende aufgeführt:

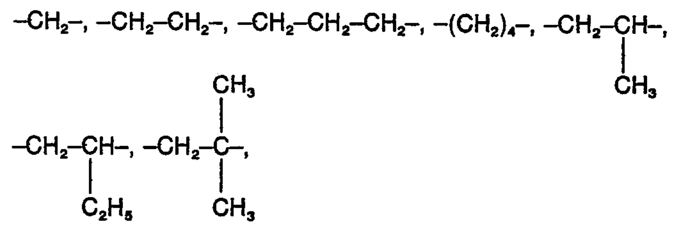

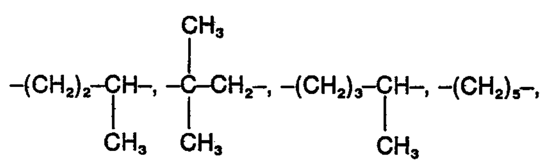

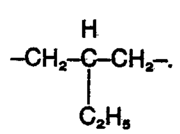

$R^1$ oder $R^2$ steht beispielsweise für Methyl, Äthyl, n- und i-Propyl, n-, i- und t.-Butyl.

Als Substituenten für substituiertes Phenyl $R^1$ und $R^2$ kommen in Frage: ($C_1$–$C_4$)-Alkyl, ($C_1$–$C_4$)-Alkoxy, Halogen (insbesondere F, Cl und Br), $CF_3$, $NO_2$ und CN.

Substituiertes Phenylalkyl $R^1$ und $R^2$ steht für durch ($C_1$–$_4$)-Alkyl, ($C_1$–$C_4$)-Alkoxy, Halogen, (insbesondere F, Cl, Br), $CF_3$, $NO_2$ und CN substituiertes Phenylalkyl, wobei der Alkylteil des Phenylalkylrestes 1 bis 4 Kohlenstoffatome besitzt. Insbesondere seien aufgeführt:

Benzyl, β-Phenyläthyl, α-Phenyläthyl, 4-Methylphenylmethyl, 3-Methoxyphenylmethyl, 4-Chlorphenylmethyl,

Falls $R^1$ der Gruppen $NHR_1$ oder $NR^1R^2$ mit einem Kohlenstoffatom des Substituenten Z zu einem 5- bis 7-gliedrigen gegebenenfalls durch ($C_1$–$C_4$)-Alkyl substituierten Ring verbunden sind, entsteht dabei ein heterocyclisches Ringsystem.

Typische Beispiele dafür sind:
1-Methyl-3-pyrrolidyl,
1-Methyl-3-piperidyl,
1-Methyl-4-piperidyl,
1-Methyl-3-hexanhydroazepinyl,
1-Methyl-4-hexahydroazepinyl.

Solche heterocyclischen Ringe sind beispielsweise
2-Methylenpyrrolidin,
2-Methyl-,
3-Methyl-,
4-Methyl-,
5-Methyl-2-methylen-pyrrolidin,
2-Methylen-1-methyl-pyrrolidin,
1,2-Dimethyl-,
1,3-Dimethyl-,
1,4-Dimethyl-,
1,5-Dimethyl-2-methylen-pyrrolidin,
2-Äthyl-2-methylen-pyrrolidin,
2-(1-Äthylen)-pyrrolidin,

2-(2-Äthylen)-pyrrolidin,
2-(1-Äthyl)-1-methyl-pyrrolidin,
(2-(1-Äthylen)-1-methyl-pyrrolidin,
2-Methylen-piperidin,
2-Methyl-2-methylen-piperidin,
2-(1-Äthylen)-piperidin,
2-(2-Äthylen)-piperidin,
2-(1-Äthylen)-2-methyl-piperidin,
2-(2-Äthylen)-2-methyl-piperidin,
1-Methyl-2-methylen-piperidin,
2-Methylen-hexahydroazepin,
2-Methyl-2-methylen-hexahydrozepin,
1,2-Dimethyl-2-methylen-hexahydrozepin.

Als gegebenenfalls durch ($C_1$–$C_4$)-Alkyl substituierte Azabicycloalkylreste $NR^1R^2$ mit 6 bis 10 Ringgliedern seien beispielhaft die folgenden aufgeführt:
2-Azabicyclo[2.2.2]octyl,
3-Azabicyclo[3.2.1]octyl,
2-Methyl-3-azabicyclo[3.2.1]octyl,
2-Äthyl-3-azabicyclo[3.2.1]octyl,
2.4-Dimethyl-3-azabicyclo[3.2.1]octyl,
1.8.8-Trimethyl-3-azabicyclo[3.2.1]octyl,
1.6.6-Trimethyl-3-azabicyclo[3.2.1]octyl,
2.2.4.4-Tetramethyl-3-azabicyclo[3.2.1]octyl,
2-Azabicyclo[3.2.1]octyl,
6-Azabicyclo[3.2.1]octyl,
8-Azabicyclo[3.2.1]octyl,
3-Azabicyclo[4.1.1]octyl,
3-Azabicyclo[3.3.0]octyl,
3-Azabicyclo[3.3.1]nonyl,
6-Methyl-3-azabicyclo[3.3.1]nonyl,
9-Azabicyclo[3.3.1]nonyl,
2-Azabicyclo[3.3.1]nonyl,
2-Azabicyclo[3.2.2]nonyl,
3-Azabicyclo[3.2.2]nonyl,
9-Azabicyclo[4.2.1]nonyl,
2-Azabicyclo[4.3.0]nonyl,
cis- und trans-8-Azabicyclo[4.3.0]nonyl,
1.6-Dimethyl-8-azabicyclo[4.3.0]nonyl,
cis- und trans-7-Azabicyclo[4.3.0]nonyl,
3-Azabicyclo[3.2.0]heptyl,
2-Methyl-3-azabicyclo[3.2.0]heptyl,
2-Azabicyclo[2.2.1]heptyl,
7-Azabicyclo[2.2.1]heptyl,
3-Azabicyclo[3.1.1]heptyl,
6-Azabicyclo[3.1.1]heptyl,
6-Azabicyclo[3.3.1]heptyl,
3-Azabicyclo[4.1.0]heptyl,
3-Azabicyclo[3.1.0]hexyl,
1-Methyl-3-azabicyclo[3.1.0]hexyl,
1,5-Dimethyl-3-azabicyclo[3.1.0]hexyl,
3-Azabicyclo[3.1.0]hexyl.

Als gegebenenfalls durch O, S, NH, $NR^1$ unterbrochene und/oder durch ein oder zwei ($C_1$–$C_4$)-Alkylgruppen substituierte Cycloalkyleniminooder Cycloalkenylimino- oder Cycloalkadienyleniminogruppen mit 4 bis 7 Ringgliedern seien beispielshaft die folgenden aufgeführt:
Pyrrolidino,
2-Methyl-,
3-Methyl-,
2,3-Dimethyl-,
2,4-Dimethyl-,
3,4-Dimethyl-pyrrolidino,
Piperidino,
2-Methyl-,
3-Methyl-,
4-Methyl-,
2,4-Dimethyl-,
2,5-Dimethyl-,
2,6-Dimethyl-,
3,4-Dimethyl-,
4-Äthyl-piperidino,
1,2,3,6-Tetrahydropiperidino,
4-Methyl-,
3,4-Dimethyl-,
4-Äthyl-,
4-Isopropyl-,
3,5-Dimethyl-1,2,3,6-tetrahydro-piperidino,
Hexahydroazepino,
2-Methyl-,
3-Methyl-,
4-Methyl-hexahydroazepino,
Morpholino,
Thiomorpholino,
Piperazino,
N-Methyl-piperazino,
N-Äthylpiperazino,
2-Methyl-,
3-Methyl-,
3,5-Dimethyl-piperazino,
2-Methylmorpholino,
2,6-Dimethylenmorpholino.

Für $R^3$ = ($C_1$–$C_4$)-Alkyl seien Methyl, Äthyl, n-Propyl, i-Propyl, n-, i-, t.-Butyl aufgeführt.

Als Alkenyl ($C_2$–$C_6$) $R^3$ seien genannt: Äthenyl, Propenyl-(1), Propenyl-(2) und Butenyl-(3).

Die erfindungsgemäss als Ausgangsprodukte einzusetzenden o-Phenylendiamin-Derivate der Formel (II) sind teilweise noch nicht bekannt. Sie können aber leicht, wie an folgendem Beispiel gezeigt, durch Umsetzung von gegebenenfalls substituierten Aminoalkanolen oder -thiolen zweckmässigerweise in Form ihrer Alkalisalze mit 5-Chlor-2-nitroanilin und nachfolgende Reduktion mit z.B. (Raney-Ni/$H_2$); (Pd-C Katalysator/$H_2$); Pt $O_2$-Kat./$H_2$) hergestellt werden, z.B.:

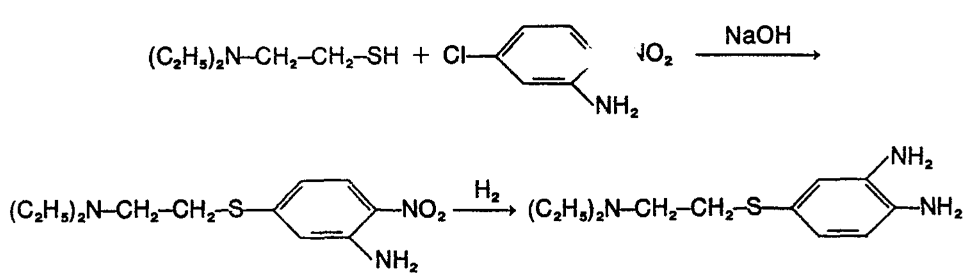

Andere Wege zu den vorgenannten Verbindungen der Formel (II) sind ebenfalls möglich. So werden 2-Nitro-5-aminoalkylmercapto-anilide mit einem Oxidationsmittel zu 2-Nitro-5-aminoalkyl-sulfinyl- oder 2-Nitro-5-aminoalkyl-sulfonyl-aniliden oxidiert, nachfolgend zu den entsprechenden Anilinen verseift und zu substituierten Sulfinyl- oder Sulfonyl-1,2-phenylendiaminen reduziert, wie an folgenden Beispielen gezeigt sei:

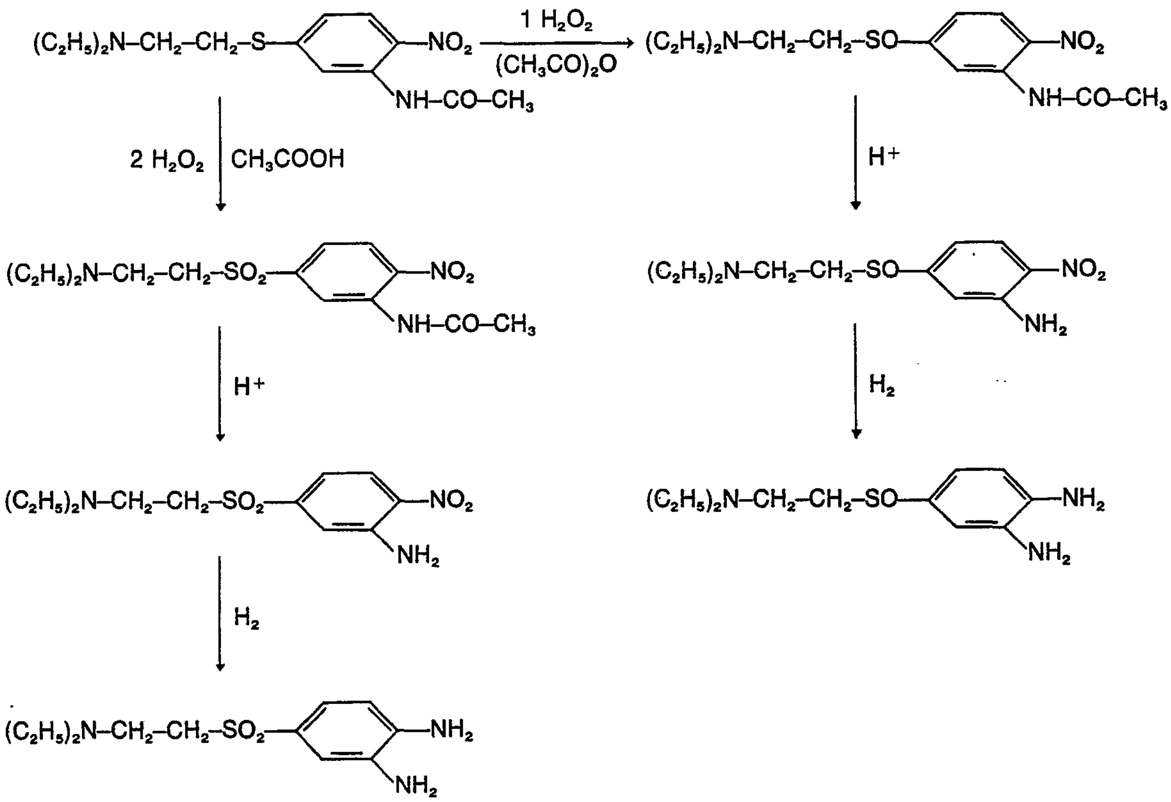


Die als Ausgangsstoffe eingesetzten Säurederivate der Formel (III) sind zum grossen Teil bekannt.

Als Ausgangsstoffe der Formel (III) seien beispielhaft genannt:

N-Methoxycarbonylcyanamid,
N-Methoxycarbonylisothioharnstoffmethyläther,
N-Methoxycarbonylisoharnstoffmethyläther,
N-Methoxycarbonylguanidin,
N-[(Bis-methoxy)methylen]-carbamidsäuremethylester,
N-[(Bis-methylmercapto)methylen]-carbamidsäuremethylester,
N-[Methoxy-(methylmercapto)-methylen]-carbamidsäuremethylester,
N.N'-Bis-methoxycarbonyl-isothioharnstoff-S-methyläther.

Unbekannte Ausgangsverbindungen der Formel (III) können nach an sich bekannten Verfahren hergestellt werden.

Die 2-Aminobenzimidazol-Derivate der Formel (IV) sind teilweise noch nicht bekannt, sie können aber leicht hergestellt werden, indem man Phenylendiaminderivate der Formel (II) mit Chlorcyan in einem inerten Lösungsmittel, gegebenenfalls unter Zusatz einer tertiären Base, wie Triäthylamin oder in einer wässrig/alkoholischen Lösung unter Zusatz einer Base, wie z.B. $NaHCO_3$ oder NaOH, miteinander umsetzt. Z.B.

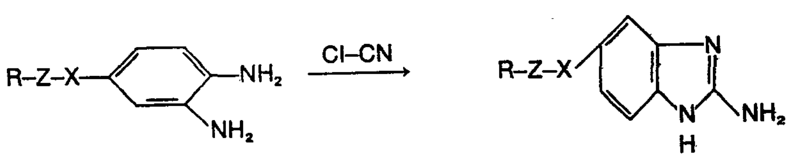


(II)

Die erfindungsgemässen Substanzen können in freier Form oder in Form ihrer physiologisch unbedenklichen Salze mit anorganischen und organischen Säuren, beispielsweise als Hydrohalogenide, vorzugsweise Hydrochloride, Sulfate, Phosphate, Nitrate, Maleinate, Fumarate, Acetate,

Methansulfonate, Hydroxylacetate, oder Naphthalindisulfonate angewendet werden.

Bei der Durchführung der Verfahrensvariante a) werden von den Säurederivaten der Formel (III) vorzugsweise
N-Methoxycarbonylcyanamid,
N-Methoxycarbonylisothioharnstoffalkyläther,
N-Methoxycarbonylisoharnstoffalkyläther,
N.N'-Bis-methoxycarbonyl-isothioharnstoff-S-methyläther,
N-Methoxycarbonylguanidin,
N-[(Bisalkoxy)methylen]-carbamidsäureester,
N-[(Bisalkylmercapto)methylen]-carbamidsäureester und
N-[Alkoxy-(alkylmercapto)-methylen]-carbamidsäureester
mit den Phenylendiaminderivaten der Formel (II) bei 0 bis 150 °C, vorzugsweise bei 30 bis 120 °C, gegebenenfalls in einem Lösungsmittel, wie Alkohol, verdünnte Essigsäure, Äthylenglykol, Tetrahydrofuran, Dioxan, Benzol, Toluol oder Wasser, umgesetzt. Vorteilhaft ist es, in Lösungsmitteln zu arbeiten, die Wasser enthalten und in denen ein pH-Bereich von 2 bis 7, vorzugsweise 2 bis 5, durch Zufügen einer organischen Säure, wie Essigsäure oder Milchsäure oder p-Toluolsulfonsäure oder eines Alkalisalzes einer organischen Säure eingehalten wird.

Die N-[(Bis-halogen)-methylen]-carbamidsäureester werden vorteilhaft in Gegenwart einer Base, wie Triäthylamin, Pyridin, Natronlauge, Natriumbicarbonat oder Natriumcarbonat mit den Phenylendiaminderivaten der Formel II bei 0 bis 100 °C, vorzugsweise 0 bis 50 °C, kondensiert.

Die N-[(Alkoxy-(halogen)-methylen]-carbamidsäureester und N-[Alkylmercapto-(halogen)-methylen]-carbamidsäureester werden vorteilhaft zunächst in Gegenwart einer Base, wie Triäthylamin, Pyridin, Natronlauge oder Natriumcarbonat bei Zimmertemperatur umgesetzt und anschliessend durch Erhitzen, vorzugsweise in einem pH-Bereich von 2 bis 5 durch Zusatz einer organischen Säure, wie Essigsäure oder Milchsäure, bei 0 bis 150 °C, vorzugsweise bei 20–120 °C, kondensiert.

Bei der Verfahrensvariante b) setzt man gegebenenfalls in Anwesenheit einer organischen Base, wie Triäthylamin, Pyridin oder einer anorganischen Base, wie einem Alkali-, Erdalaklialkoholat, z. B. Natriummethylat oder Alkali- oder Erdalkalihydroxid, z. B Natriumhydroxid, bei Temperaturen zwischen etwa 20 und etwa 150 °C, vorzugsweise zwischen 60 und 120 °C, in einem für diese Umsetzung indifferentem organischen Lösungsmittel, wie z. B. Tetrahydrofuran, Dioxan, Benzol, Toluol, Chlorbenzol, Acetonitril, Aceton, Methyläthylketon, Diäthylenglykoldimethyläther, Methanol und Äthanol oder Gemischen der vorgenannten Verdünnungsmittel um.

Bei der Reaktionsvariante c) setzt man 1 Mol der Verbindung (VI) mit einem oder mindestens 1 Mol des Oxidationsmittels nach an sich bekannten Verfahrensbedingungen in einem inerten organischen Lösungsmittel oder Lösungsmittelgemisch, beispielsweise in Wasser, Essigsäure, Ameisensäure, Acetanhydrid, Propionanhydrid, bei Temperaturen zwischen etwa 0 und 100 °C, vorzugsweise zwischen 0 und 80 °C, um. Die Aufarbeitung geschieht nach üblichen Methoden.

Die bei der Verfahrensvariante c) als Ausgangsprodukte eingesetzten Oxidationsmittel sind bereits bekannt.

Beispielhaft seien genannt:

Organische Persäuren, wie Peressigsäure, Perameisensäure, Perbenzoesäure, m-Chlorperbenzoesäure, Monoperphthalsäure, anorganische Peroxide, wie Wasserstoffperoxid, gelöst in Wasser oder ggf. verdünnten organischen Säuren, anorganische Oxidationsmittel, wie Chromsäure, Salpetersäure, Kaliumpermanganat, Chlor, Brom, Halogensauerstoffsäuren, wie unterchlorige, chlorige, Chlor- oder Überchlorsäure, tert.-Butylhypochlorit, Methylhypochlorit, tert.-Butylchromat, organische N-Halogenverbindungen, wie N-Chlorsuccinimid, N-Bromsuccinimid sowie N-Halogensulfonsäureamide oder N-Halogencarbonsäureamide.

Durch entsprechend literaturbekannte Wahl der Reaktionsbedindungen kann das Oxidationspotential entsprechend eingestellt und die Reaktion zur Herstellung der Sulfoxide bzw. Sulfone gelenkt werden.

Als erfindungsgemässe Wirkstoffe seien aufgeführt:
5-(2-Diäthylaminoäthylthio)-benzimidazolyl-2-carbamidsäuremethylester,
5-(2-Dimethylaminoäthylthio)-benzimidazolyl-2-carbamidsäuremethylester,
5-(3-Dimethylaminopropylthio)-benzimidazolyl-2-carbamidsäuremethylester,
5-(2-Dimethylaminopropylthio)-benzimidazolyl-2-carbamidsäuremethylester,
5-(2-Morpholinoäthylthio)-benzimidazolyl-2-carbamidsäuremethylester,
5-(2-Morpholinopropylthio)-benzimidazolyl-2-carbamidsäuremethylester
sowie die in den nachfolgenden Beispielen aufgeführten Verbindungen der allgemeinen Formel (I).

Die erfindungsgemässen Verbindungen besitzen eine ausgezeichnete Wirksamkeit gegenüber Helminthen, insbesondere eine überraschend gute und breite Wirkung gegen folgende Nematoden und Cestoden:

1. Hakenwürmer (z. B. Bunostomum trigonocephalum, Uncinaria stenocephala);
2. Trichostrongyliden (z. B. Haemonchus contortus, Trichostrongylus colubriformis, Ostertagia circumcincta, Nippostrongylus muris, Cooperia curticei);
3. Strongyliden (z. B. Oesophagostomum columbianum);
4. Rhabditiden (z. B. Strongyloides ratti);
5. Spulwürmer (z. B. Toxcara canis, Toxascaris leonina, Ascaris suum);
6. Madenwürmer (z. B. Aspiculuris tetraptera);
7. Heterakiden (z. B. Heterakis spumosa);
8. Peitschenwürmer (z. B. Trichuris muris);
9. Filarien (z. B. Litomosoides carinii, Dipetalonema witei);

10. Bandwürmer (z. B. Taenis pisiformis, Hymenolepis nana).

Die Wirkung wurde im Tierversuch nach oraler und parenteraler Applikation bei stark mit Parasiten befallenen Versuchstieren geprüft. Die angewendeten Dosierungen wurden sehr gut von den Versuchstieren vertragen.

Die neuen Wirkstoffe können als Anthelmintika sowohl in der Human- als auch in der Veterinärmedizin verwendet werden.

Die neuen Wirkstoffe können in bekannter Weise in die üblichen Formulierungen übergeführt werden.

Die neuen Verbindungen können in den Formulierungen auch in Mischungen mit anderen bekannten Wirkstoffen vorliegen.

Die neuen Wirkstoffe können in üblicher Weise angewendet werden. Die Applikation erfolgt vorzugsweise parenteral, insbesondere subcutan, jedoch sind beispielsweise auch eine orale oder eine dermale («Pour-on») Applikation ebenfalls möglich.

Im allgemeinen hat es sich als vorteilhaft erwiesen, Mengen von etwa 1 bis 100 mg der neuen Verbindungen je kg Körpergewicht pro Tag zur Erzielung wirksamer Ergebnisse zu verabreichen.

Trotzdem kann es gegebenenfalls erforderlich sein, von den genannten Mengen abzuweichen, und zwar in Abhängigkeit von dem Körpergewicht des Versuchstieres und der Art des Applikationsweges, aber auch aufgrund der Tierart und deren individuellem Verhalten gegenüber dem Medikament bzw. der Art, von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Fall der Applikation grösserer Mengen kann es empfehlenswert sein, diese in mehrere Einzelgaben über den Tag zu verteilen. Für die Applikation in der Human- und Veterinärmedizin ist der gleiche Dosierungsspielraum vorgesehen. Sinngemäss gelten auch die weiteren obigen Ausführungen.

Beispiel 1

5-(2-Diäthylaminoäthylthio)-benzimidazolyl-2-carbamidsäure-methylester

Zu 7,1 g (0,03 Mol) 4-(2-Diäthylaminoäthylthio)-1,2-phenylendiamin, gelöst in 100 ml Chloroform, werden 10 ml Triäthylamin und 5,6 g (0,031 Mol) N-[(Bis-chlor)-methylen]-carbamidsäuremethylester bei 20 °C zugetropft. Man erhitzt 30 Minuten unter Rückfluss. Nach dem Abdestillieren des Lösungsmittels im Vakuum wird der Rückstand mehrfach mit Wasser verrieben, abgesaugt, nochmals gut mit n-Hexan verrieben und abermals abgesaugt. Ausbeute: 7,9 g 5-(2-Diäthylaminoäthylthio)-benzimidazolyl-2-carbamidsäure-methylester. Fp. 162–164 °C (Zers.), Hydrochlorid Fp. 200–201 °C (Zers.).

Das als Ausgangsmaterial verwendete 4-(2-Diäthylaminoäthylthio)-1,2-phenylendiamin, Öl, wird durch katalytische Hydrierung von 2-Nitro-5-(2-diäthylaminoäthylthio)-anilin erhalten. Das 2-Nitro-5-(2-diäthylaminoäthylthio)-anilin (Fp. 42–44 °C) seinerseits wird durch Umsetzung von 2-Diäthylaminoathanthiol in Gegenwart von Natriummethylat in Alkohol mit 5-Chlor-2-nitroanilin hergestellt.

Beispiel 2

N-Methoxycarbonyl-isothioharnstoffmethyläther, der durch Umsetzung von 7,8 g S-Methylisothioharnstoffsulfat mit 4,5 g Chlorameisensäuremethylester und 17 g 25%iger Natronlauge gewonnen wurde, wird mit Essigsäure (~5–6 ml) auf pH 5 gebracht. Man setzt 7,1 g 4-(2-Diäthylaminoäthylthio)-1,2-phenylendiamin, gelöst in Alkohol und 3 ml Essigsäure, zu, erhitzt 1 Stunde auf 80–85 °C, kühlt ab, filtriert das Reaktionsprodukt ab und erhält nach Waschen mit Wasser und Alkohol 9 g 5-(2-Diäthylaminoäthylthio)-benzimidazolyl-carbamidsäuremethylester vom Fp. 162–164 °C.

Die gleiche Verbindung erhält man analog durch Kondensation mit N-Methoxycarbonyl-isoharnstoffmethyläther in einer Menge von 4,2 g oder durch Kondensation von 4-(2-Diäthylaminoäthylthio)-1,2-phenylendiamin (0,03 Mol) mit 0,03 Mol N-[(Bis-äthoxy)-methylen]-carbamidsäuremethylester bei 110 bis 140 °C in Glykol (Ausbeute 5,2 g) oder mit 0,03 Mol N-[(Bis-alkylmercapto)-methylen]-carbamidsäuremethylester (Ausbeute 4,9 g) oder mit 0,03 Mol N-[Äthoxy-(methylmercapto)-methylen]-carbamidsäuremethylester (Ausbeute 4,1 g) oder mit 0,03 Mol N-Methoxycarbonylguanidin bei 140 bis 160 °C (Ausbeute 3,9 g) oder mit 0,03 Mol Methoxycarbonylcyanamid in Wasser bei 80 bis 100 °C, wobei mit Salzsäure ein pH von 2 bis 4 eingestellt wird (Ausbeute 3,8 g), oder mit 0,03 Mol N,N'-Bis-methoxycarbonylisothioharnstoff-S-methyläther (Ausbeute 8,3 g).

Beispiel 3

Zu 24,8 g (0,1 Mol) 4-(2-Diäthylaminoäthylthio)-1,2-phenylendiamin und 10,1 g (0,1 Mol) Triäthylamin in 300 ml trockenem Chloroform werden unter Rühren bie 0 °C 16,7 g (0,1 Mol) N-[Chlor-(methylmercapto)-methylen]-carbamidsäuremethylester, gelöst in wenig Chloroform, zugetropft. Man rührt 2 bis 3 Stunden bei 25 bis 30 °C nach, dampft im Vakuum ein, fügt 200 ml Alkohol, 20 ml Wasser und 0,5 g p-Toluolsulfonsäure zu und erhitzt 2 Stunden auf 90 °C. Nach dem Eindampfen verrührt man den Rückstand mit Wasser, viltriert ab und wäscht das Reaktionsprodukt mit Alkohol. Ausbeute 26,2 g 5-(2-Diäthylaminoäthylthio)-benzimidazolyl-2-carbamidsäuremethylester. Kondensiert man analog 0,1 Mol N-[Chlor-(methoxy)methylen]-carbamidsäuremethylester mit 4-(2-Diäthylaminoäthylthio)-1,2-phenylendiamin, so erhält man eine Ausbeute von 18,5 g.

Beispiel 4

27,3 g (0,1 Mol) 2-Amino-5-(2-diäthylaminoäthylthio)-benzimiazol fügt man zu einer Lösung von 2,4 g Natrium in 200 ml Methanol, tropft 5,6 g

Kohlensäuredimethylester ein und erhitzt eine Stunde unter Rückfluss. Man dampft im Vakuum ein, wäscht den Rückstand nacheinander mit Wasser, verdünnter Essigsäure und Alkohol und erhält nach dem Trocknen 21,4 g 5-(2-Diäthylaminoäthylthio)-benzimidazolyl-2-carbamidsäuremethylester, Fp. 162–164 °C.

Die gleiche Verbindung entsteht durch Umsetzung von 2-Amino-5-(2-diäthylaminoäthylthio)-benzimidazol mit einer äquivalenten Menge Pyrokohlensäuredimethylester in Chloroform bei 40 °C.

Die gleiche Verbindung entsteht ebenso durch Umsetzung von 0,1 Mol 2-Amino-5-(2-diäthylaminoäthylthio)-benzimidazol, gelöst in Chloroform, mit 0,1 Mol Chlorameisensäuremethylester in Gegenwart von 0,1 Mol Triäthylamin. Nach beendetem Eintropfen erhitzt man noch 5 bis 6 Stunden unter Rückfluss. Ausbeute: 22,6 g.

Das als Ausgangsmaterial verwendete 2-Amino-5-(2-diäthylaminoäthylthio)-benzimidazol entsteht durch Umsetzung von 4-(2-Diäthylaminoäthylthio)-1,2-phenylendiamin mit Chlor- oder Bromcyan in Tetrahydrofuran, Alkohol oder Wasser.

Beispiel 5

Nach der in Beispiel 1 beschriebenen Darstellung erhält man aus 7,1 g 4-(Diäthylaminoäthylsulfinyl)-1,2-phenylendiamin und 5,6 g N-[(Bis-chlor)-methylen]-carbamidsäuremethylester 7,2 g 5-(2-Diäthylaminoäthylsulfinyl)-benzimidazolylcarbamidsäuremethylester.

Das als Ausgangsmaterial verwendete 4-(2-Diäthylaminoäthylsulfinyl)-1,2-phenylendiamin wird auf folgendem Wege erhalten: 2-Nitro-5-(2-diäthylaminoäthylthio)-propionanilid wird in Acetanhydrid mit einem Äquivalent $H_2O_2$ zu 2-Nitro-5-(2-diäthylaminoäthylsulfinyl)-propionanilid oxidiert, nachfolgend zum 2-Nitro-5-(2-diäthylaminoäthylsulfinyl)-anilin verseift und katalytisch zum 5-(2-Diäthylaminoäthylsulfinyl)-1,2-phenylendiamin reduziert.

Beispiel 6

12,8 g 5-(2-Diäthylaminoäthylthio)-benzimidazol-2-carbamidsäuremethylester werden in 500 ml Acetanhydrid gelöst. Dazu tropft man 4,7 g 30%iger Wasserstoffsuperoxidlösung, rührt 3 Stunden nach und dampft im Vakuum ein. Der Rückstand wird mit einem Gemisch von Petroläther/Essigester verrieben. Nach dem Abfiltrieren erhält man 5-(2-Diäthylaminoäthylsulfinyl)-benzimidazolyl-2-carbamidsäuremethylester (Zersetzung).

In entsprechender Arbeitsweise erhält man aus 12,8 g 5-(2-Diäthylaminoäthylthio)-benzimidazolyl-2-carbamidsäuremethylester, gelöst in 250 ml Essigsäure mit 12 g 30%igem Wasserstoffperoxid 6 g 5-(2-Diäthylaminoäthylsulfonyl)-benzimidazolyl-2-carbamidsäuremethylester.

Beispiele 7 bis 159

Nach der in Beispiel 1 beschriebenen Methode erhält man aus substituierten Phenylendiamin-Derivaten folgende Benzimidazolyl-2-carbamidsäureester:

| Nr. | Ausgangsstoffe 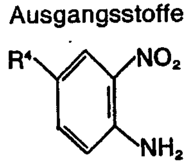 F°C | 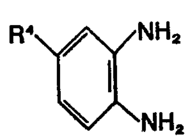 Kp°C (mm Hg) | Erfindungsgemäss hergestellte bzw. herstellbare Verbindungen 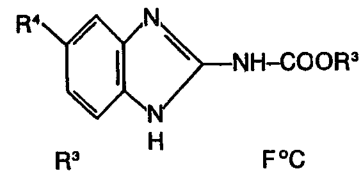 R⁴ | R³ | F°C | Salz, F°C |
|---|---|---|---|---|---|---|
| 7 | 66–68 | 178–183 (0,1) | $(CH_3)_2N-CH_2-CH_2-S$ | $CH_3$ | 185–186 (Zers.) | HCl 155–156 (Zers.) |
| 8 | 68–70 | 190–195 (0,2) | $(CH_3)_2N-CH_2-CH_2-CH_2-S$ | $CH_3$ | 184–185 (Zers.) | HCl 160–162 (Zers.) |
| 9 | 66–68 | 190–200 (0,1) | $(C_2H_5)_2N-CH_2-CH_2-CH_2-S$ | $CH_3$ | 155–156 (Zers.) | |
| 10 | 104–105 | 185–190 (0,1) | 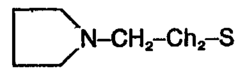 | $CH_3$ | 186–187 (Zers.) | HCl 171–172 (Zers.)<br>2HCl 182–183 (Zers.) |
| 11 | Öl | 190–200 (0,1) | 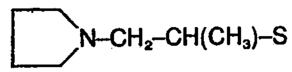 | $CH_3$ | 193–195 (Zers.) | HCl 198–200 (Zers.) |
| 12 | 115–116 | 195–200 (0,1) | 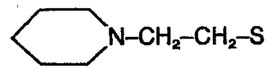 | $CH_3$ | 208–210 (Zers.) | · 2HCl · $2H_2O$,<br>110–112 (Zers.) |
| 13 | 150–152 | 195–200 (0,1) | 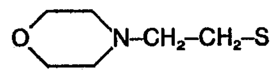 | $CH_3$ | 175–176 (Zers.) | |
| 14 | 93–95 | 190–200 (0,1) | 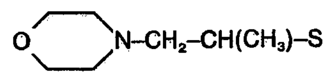 | $CH_3$ | 180–182 (Zers.) | |
| 15 | 87–90 | 200–210 (0,1) | 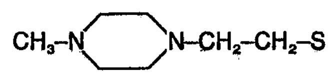 | $CH_3$ | 165–166 (Zers.) | |
| 16 | Öl | 200–210 (0,1) | 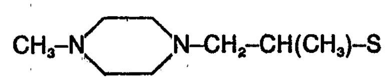 | $CH_3$ | 158–160 (Zers.) | |
| 17 | 72 | 190–195 (0,1) | $C_4H_9-NH-CH_2-CH_2-S$ | $CH_3$ | 156 (Zers.) | HCl 175–176 (Zers.) |
| 18 | Öl | 200–205 (0,1) | 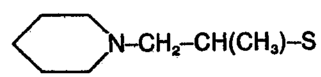 | $CH_3$ | 181–183 (Zers.) | |
| 19 | | | $(CH_3)_2N-CH_2-CH(CH_3)-CH_2-S$ | $CH_3$ | | |
| 20 | | | $(CH_3)_2N-C(CH_3)_2-CH_2-S$ | $CH_3$ | | |
| 21 | | | $(CH_3)_2N-(CH_2)_4-S$ | $CH_3$ | | |
| 22 | | 176–180 (0,2) | $(CH_3)_2N-CH_2-CH(CH_3)-S$ | $CH_3$ | 171–172 (Zers.) | |
| 23 | | | $(CH_3)_2N-(CH_2)_5-S$ | $CH_3$ | | |
| 24 | | | $(CH_3)_2N-CH(CH_3)-CH_2-S$ | $CH_3$ | | |
| 25 | | | $(CH_3)_2N-CH(CH_3)-CH_2CH_2-S$ | $CH_3$ | | |
| 26 | | | $(CH_3)_2N-CH_2-C(CH_3)_2-CH_2-S$ | $CH_3$ | | |

| Nr. | Ausgangsstoffe 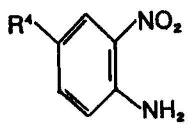 F °C | 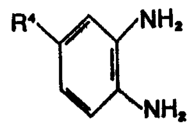 Kp °C (mm Hg) | Erfindungsgemäss hergestellte bzw. herstellbare Verbindungen R⁴ | 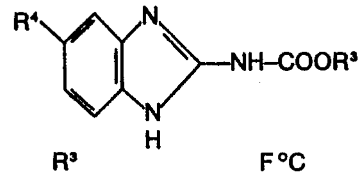 R³ | F °C | Salz, F °C |
|---|---|---|---|---|---|---|
| 27 | | | $(CH_3)_2N-CH_2CH_2CH(CH_3)-S$ | $CH_3$ | | |
| 28 | | | $(CH_3)_2N-CH_2-CH(CH_3)-CH_2-S$ | $CH_3$ | | |
| 29 | | | $(C_2H_5)_2N-(CH_{12})_4-S$ | $CH_3$ | | |
| 30 | | | $(C_2H_5)_2N-(CH_2)_5-S$ | $CH_3$ | | |
| 31 | | | $(C_2H_5)_2N-CH_2-CH(CH_3)-S$ | $CH_3$ | 175 (Zers.) | |
| 32 | | | $(C_2H_5)_2N-C(CH_3)_2-CH_2-S$ | $CH_3$ | | |
| 33 | | | $(C_2H_5)_2N-CH(CH_3)-CH_2-S$ | $CH_3$ | | |
| 34 | | | $(C_2H_5)_2N-CH(CH_3)-CH_2CH_2-S$ | $CH_3$ | | |
| 35 | | | $(C_2H_5)_2N-CH_2CH(CH_3)-CH_2-S$ | $CH_3$ | | |
| 36 | Öl | 195–200 (0,1) | $(C_2H_5)_2N-(CH_2)_3-CH(CH_3)-S$ | $CH_3$ | 180 (Zers.) | |
| 37 | | | $(C_3H_7)_2N-(CH_2)-S$ | $CH_3$ | | |
| 38 | | | $(CH_3)_2CHN-(CH_2)_2-S$ | $CH_3$ | | |
| 39 | | | $(CH_3)_2CHN-(CH_2)_3-S$ | $CH_3$ | | |
| 40 | | | $(C_4H_9)_2N-(CH_2)_2-S$ | $CH_3$ | | |
| 41 | | | $(C_4H_9)_2N-(CH_2)_3-S$ | $CH_3$ | | |
| 42 | | | $CH_3(C_2H_5)N-(CH_2)_2-S$ | $CH_3$ | | |
| 43 | | | $CH_3(C_2H_5)N-(CH_2)_3-S$ | $CH_3$ | | |
| 44 | | | $C_6H_5CH_2(CH_3)N-(CH_2)_2-S$ | $CH_3$ | | |
| 45 | Öl | Öl | $C_6H_5CH_2(CH_3)N-CH_2-CH(CH_3)-S$ | $CH_3$ | 159–161 (Zers.) | |
| 46 | | | $C_6H_5(CH_3)N-(CH_2)_2-S$ | $CH_3$ | | |
| 47 | | | $C_6H_5(CH_3)N-(CH_2)_3-S$ | $CH_3$ | | |
| 48 | | | 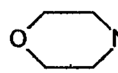 N–$(CH_2)_3$–S | $CH_3$ | | |
| 49 | | | 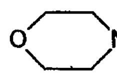 N–$CH_2$–$CH(CH_3)$–$CH_2$–S | $CH_3$ | | |
| 50 | | | 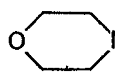 N–$C(CH_3)_2$–$CH_2$–S | $CH_3$ | | |
| 51 | | | 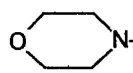 N–$(CH_2)_4$–S | $CH_3$ | | |
| 52 | | | 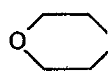 N–$(CH_2)_5$–S | $CH_3$ | | |

| Nr. | Ausgangsstoffe 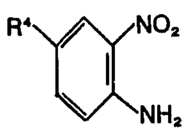 F °C | 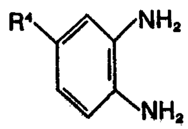 Kp °C (mm Hg) | Erfindungsgemäss hergestellte bzw. herstellbare Verbindungen 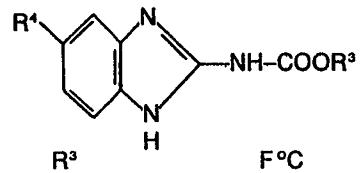 $R^4$ | $R^3$ | F °C | Salz, F °C |
|---|---|---|---|---|---|---|
| 53 | | | 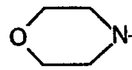$-CH(CH_3)-CH_2-S$ | $CH_3$ | | |
| 54 | | | 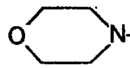$-CH(CH_3)-CH(CH_3)-S$ | $CH_3$ | | |
| 55 | | | 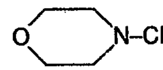$-CH(CH_3)-CH_2-CH_2-S$ | $CH_3$ | | |
| 56 | | | 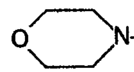$-CH_2-CH(CH_3)-CH_2-S$ | $CH_3$ | | |
| 57 | | | 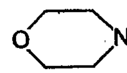$-CH_2-CH_2-CH(CH_3)-S$ | $CH_3$ | | |
| 58 | | | 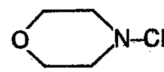$-CH_2-C(CH_3)_2-CH_2-S$ | $CH_3$ | | |
| 59 | | | 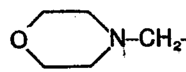$-CH_2-C(C_2H_5)-CH_2-S$ | $CH_3$ | | |
| 60 | | | 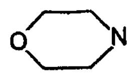$-(CH_2)_2-C(CH_3)_2-S$ | $CH_3$ | | |
| 61 | | | 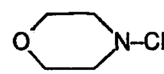$-CH_2-C(C_2H_5)-S$ | $CH_3$ | | |
| 62 | | | 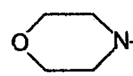$-CH_2-C(C_3H_7)-S$ | $CH_3$ | | |
| 63 | | | 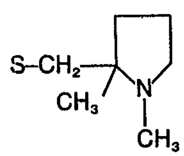 | $CH_3$ | | |

| Nr. | Ausgangsstoffe<br>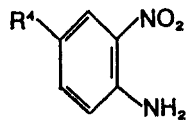<br>F°C | 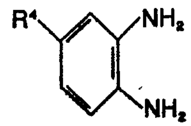<br>Kp°C (mm Hg) | Erfindungsgemäss hergestellte bzw. herstellbare Verbindungen<br>$R^4$ | 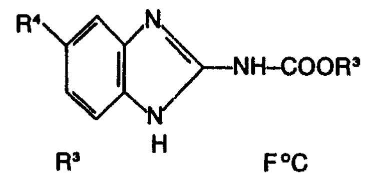<br>$R^3$ | F°C | Salz, F°C |
|---|---|---|---|---|---|---|
| 64 | | | 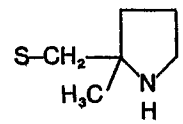 | $CH_3$ | | |
| 65 | | | 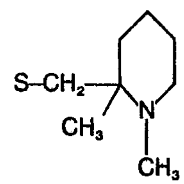 | $CH_3$ | | |
| 66 | | | 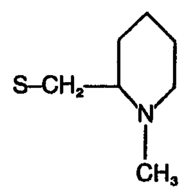 | $CH_3$ | | |
| 67 | | | 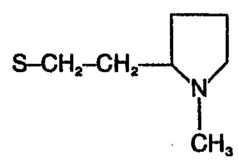 | $CH_3$ | | |
| 68 | | | 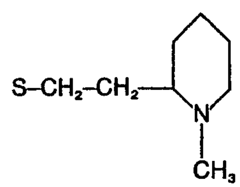 | $CH_3$ | | |
| 69 | | | 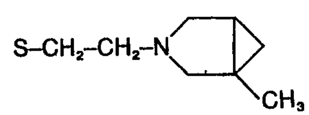 | $CH_3$ | | |

| Nr. | Ausgangsstoffe 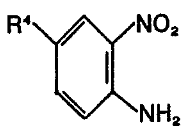 F°C | 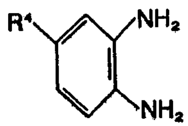 Kp°C (mm Hg) | Erfindungsgemäss hergestellte bzw. herstellbare Verbindungen R⁴ | 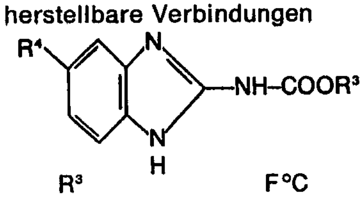 $R^3$ | F°C | Salz, F°C |
|---|---|---|---|---|---|---|
| 70 | | | 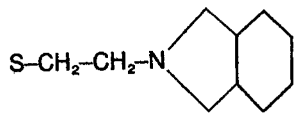 | $CH_3$ | | |
| 71 | | | 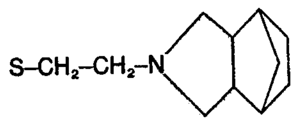 | $CH_3$ | | |
| 72 | | | 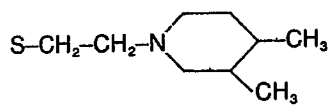 | $CH_3$ | | |
| 73 | | | 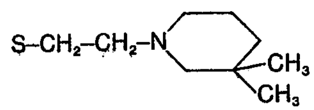 | $CH_3$ | | |
| 74 | | | 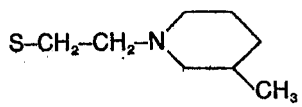 | $CH_3$ | | |
| 75 | | | 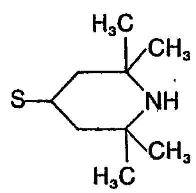 | $CH_3$ | | |
| 76 | | | 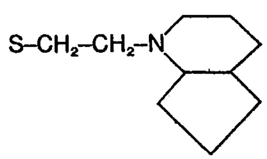 | $CH_3$ | | |

| | Ausgangsstoffe | | Erfindungsgemäss hergestellte bzw. herstellbare Verbindungen | | | |
|---|---|---|---|---|---|---|
| | 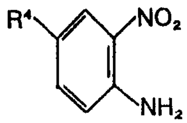 | 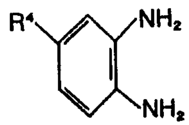 | | 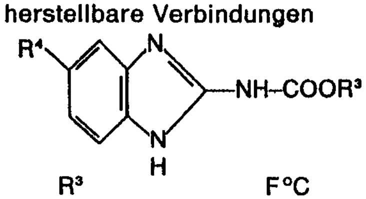 | | |
| Nr. | F°C | Kp°C (mm Hg) | $R^4$ | $R^3$ | F°C | Salz, F°C |
| 77 | | | 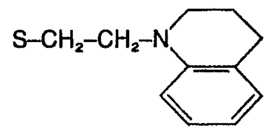 | $CH_3$ | | |
| 78 | | | $S-CH_2CH_2N(CH_3)CH_2CH_2C_6H_5$ | $CH_3$ | | |
| 79 | | | $S-CH_2-CH_2-N(CH_3)C_6H_5$ | $CH_3$ | | |
| 80 | | | 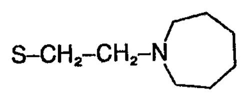 | $CH_3$ | | |
| 81 | | | 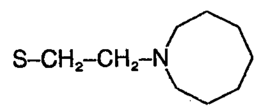 | $CH_3$ | | |
| 82 | | | 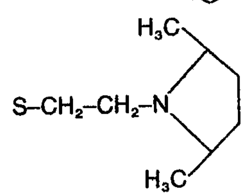 | $CH_3$ | | |
| 83 | | | 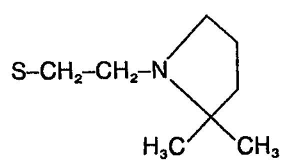 | $CH_3$ | | |
| 84 | | | 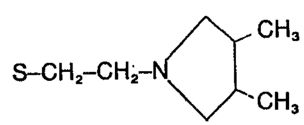 | $CH_3$ | | |

| | Ausgangsstoffe | | Erfindungsgemäss hergestellte bzw. herstellbare Verbindungen | | | |
|---|---|---|---|---|---|---|
| | 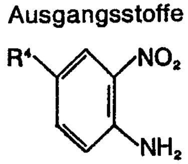 | 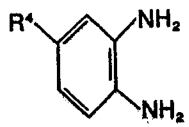 | 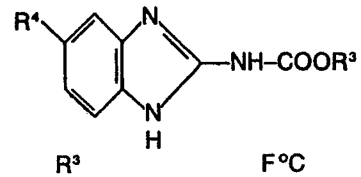 | | | |
| Nr. | F°C | Kp°C (mm Hg) | $R^4$ | $R^3$ | F°C | Salz, F°C |
| 85 | | | $S-CH_2-CH_2-$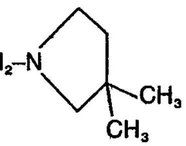 | $CH_3$ | | |
| 86 | | | $S-CH_2-CH_2-$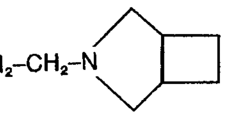 | $CH_3$ | | |
| 87 | | | $C_6H_5NH-(CH_2)_2-S$ | $CH_3$ | | |
| 88 | | | $C_6H_5NH-(CH_2)_3-S$ | $CH_3$ | | |
| 89 | | | $C_2H_5NH-(CH_2)_2-S$ | $CH_3$ | | |
| 90 | | | $C_2H_5NH-(CH_2)_3-S$ | $CH_3$ | | |
| 91 | | | $C_3H_7NH-(CH_2)_2-S$ | $CH_3$ | | |
| 92 | | | $(CH_3)_2CH-NH-(CH_2)_2-S$ | $CH_3$ | | |
| 93 | | | $(CH_3)_2CH-NH-(CH_2)_3-S$ | $CH_3$ | | |
| 94 | | | $(CH_3)_2CH-CH_2-NH-(CH_2)_2-S$ | $CH_3$ | | |
| 95 | | | $(CH_3)_2CH-CH_2-NH-(CH_2)_3-S$ | $CH_3$ | | |
| 96 | | | $(CH_3)_2CH-(CH_2)_2-NH-(CH_2)_2-S$ | $CH_3$ | | |
| 97 | | | $(CH_3)_3C-NH-(CH_2)_2-S$ | $CH_3$ | | |
| 98 | | | $(CH_3)_3C-NH-(CH_2)_3-S$ | $CH_3$ | | |
| 99 | | | $(CH_3)_2CH(CH_2)_2-NH-(CH_2)_2-S$ | $CH_3$ | | |
| 100 | | | $S-CH_2-CH_2-$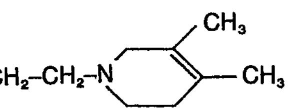 | $CH_3$ | | |
| 101 | | | $S-CH_2-CH_2-$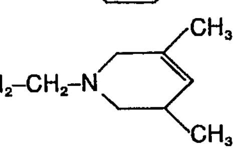 | $CH_3$ | | |
| 102 | | | $S-CH_2-CH_2-$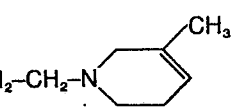 | $CH_3$ | | |

Ausgangsstoffe

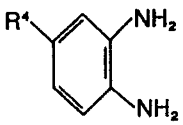 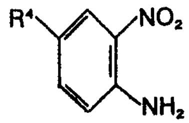

Erfindungsgemäss hergestellte bzw. herstellbare Verbindungen

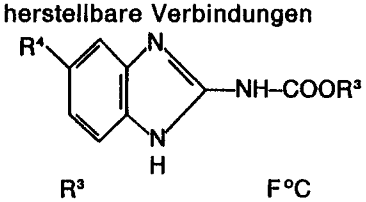

| Nr. | F°C | Kp°C (mm Hg) | $R^4$ | $R^3$ | F°C | Salz, F°C |
|---|---|---|---|---|---|---|
| 103 | | | 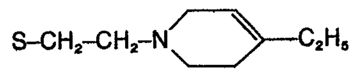 | $CH_3$ | | |
| 104 | | | 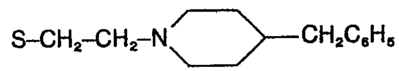 | $CH_3$ | | |
| 105 | | | 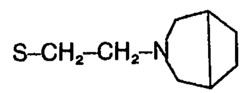 | $CH_3$ | | |
| 106 | | | 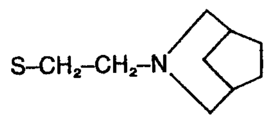 | $CH_3$ | | |
| 107 | | | 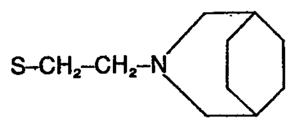 | $CH_3$ | | |
| 108 | | | 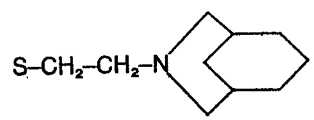 | $CH_3$ | | |
| 109 | | | 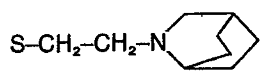 | $CH_3$ | | |
| 110 | | | $(CH_3)_2N-CH_2-CH_2-S$ | $C_2H_5$ | | |
| 111 | | | $(CH_3)_2N-CH_2-CH_2-CH_2-S$ | $C_3H_7$ | | |
| 112 | | | $(C_2H_5)_2N-CH_2-CH_2-CH_2-S$ | $C_2H_5$ | | |
| 113 | | | 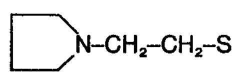 | $C_4H_9$ | | |
| 114 | | | 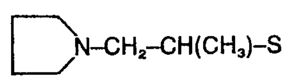 | $C_2H_5$ | | |

| Nr. | Ausgangsstoffe<br>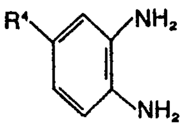<br>F°C | 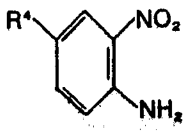<br>Kp°C (mm Hg) | Erfindungsgemäss hergestellte bzw. herstellbare Verbindungen<br>R⁴ | 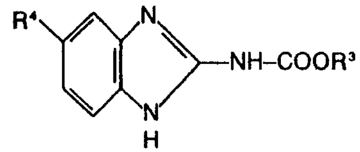<br>$R^3$ | F°C | Salz, F°C |
|---|---|---|---|---|---|---|
| 115 | | | 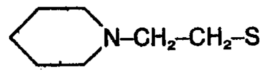 | $CH_2-CH=CH_2$ | | |
| 116 | | | 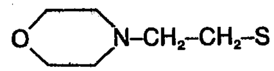 | $CH_2-CH=CH_2$ | | |
| 117 | | | 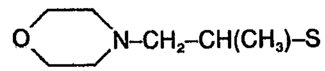 | $C_2H_5$ | | |
| 118 | | | 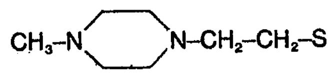 | $C_2H_5$ | | |
| 119 | | | 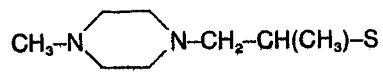 | $C_2H_5$ | | |
| 120 | | | $C_4H_9-\underset{H}{N}-CH_2-CH_2-S$ | $C_2H_5$ | | |
| 121 | | | 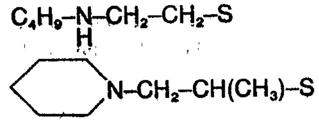 | $C_2H_5$ | | |
| 122 | | | $(CH_3)_2N-CH_2-CH_2-O$ | $CH_3$ | | |
| 123 | | | $(CH_3)_2N-CH_2-CH_2-CH_2-O$ | $CH_3$ | | |
| 124 | | | $(C_2H_5)_2N-CH_2-CH_2-CH_2-O$ | $CH_3$ | | |
| 125 | | | 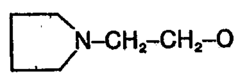 | $CH_3$ | | |
| 126 | | | 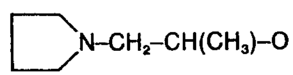 | $CH_3$ | | |
| 127 | | | 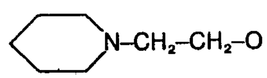 | $CH_3$ | | |
| 128 | 107–109 | 185–195 (0,2) | 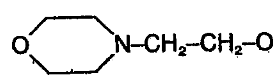 | $CH_3$ | 204–205 (Zers.) | |

| Nr. | Ausgangsstoffe 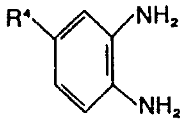 F°C | 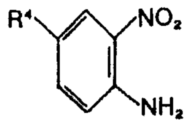 Kp°C (mm Hg) | Erfindungsgemäss hergestellte bzw. herstellbare Verbindungen 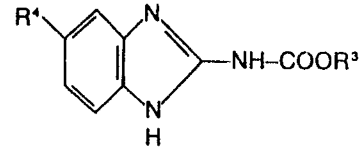 $R^4$ | $R^3$ | F°C | Salz, F°C |
|---|---|---|---|---|---|---|
| 129 | | | 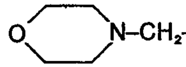$-CH_2-CH(CH_3)-O$ | $CH_3$ | | |
| 130 | 117 | Öl | $C_6H_5(CH_3)N-CH_2-CH_2-O$ | $CH_3$ | 222 (Zers.) | |
| 131 | | | $C_6H_5NH-CH_2-CH_2-O$ | $CH_3$ | | |
| 132 | | | 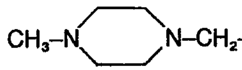$-CH_2-CH_2-O$ | $CH_3$ | | |
| 133 | 65–67 | 170–180 (0,2) | $(C_2H_5)N-CH_2-CH_2-O$ | $CH_3$ | 190–191 (Zers.) | |
| 134 | | | $C_4H_9-NH-CH_2-CH_2-O$ | $CH_3$ | | |
| 135 | | | 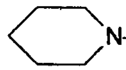$-CH_2-CH(CH_3)-O$ | $CH_3$ | | |
| 136 | | | $(CH_3)_2N-CH_2-CH_2-SO$ | $CH_3$ | | |
| 137 | | | $(CH_3)_2N-CH_2-CH_2-CH_2-SO$ | $CH_3$ | | |
| 138 | | | $(C_2H_5)_2N-CH_2-CH_2-CH_2-SO$ | $CH_3$ | | |
| 139 | | | 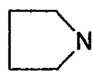$-CH_2-CH_2-SO$ | $CH_3$ | | |
| 140 | | | 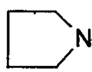$-CH_2-CH(CH_3)-SO$ | $CH_3$ | | |
| 141 | | | 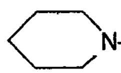$-CH_2-CH_2-SO$ | $CH_3$ | | |
| 142 | | | 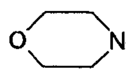$-CH_2-CH_2-SO$ | $CH_3$ | | |
| 143 | | | 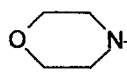$-CH_2-CH(CH_3)-SO$ | $CH_3$ | | |
| 144 | | | 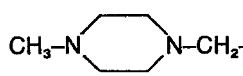$-CH_2-CH_2-SO$ | $CH_3$ | | |

| Nr. | Ausgangsstoffe 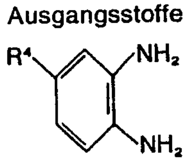 F°C | 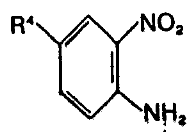 Kp°C (mm Hg) | Erfindungsgemäss hergestellte bzw. herstellbare Verbindungen 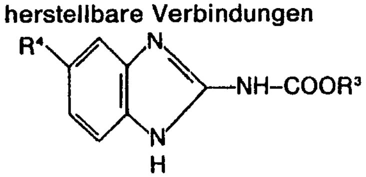 R⁴ | R³ | F°C | Salz, F°C |
|---|---|---|---|---|---|---|
| 145 | | | 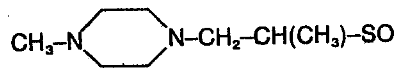 | $CH_3$ | | |
| 146 | | | $C_4H_9-NH-CH_2-CH_2-SO$ | $CH_3$ | | |
| 147 | | | 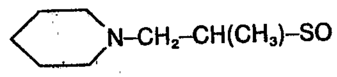 | $CH_3$ | | |
| 148 | | | $(CH_3)_2N-CH_2-CH_2-SO_2$ | $CH_3$ | | |
| 149 | | | $(CH_3)_2N-CH_2-CH_2-CH_2-SO_2$ | $CH_3$ | | |
| 150 | | | $(C_2H_5)_2N-CH_2-CH_2-CH_2-SO_2$ | $CH_3$ | | |
| 151 | | | 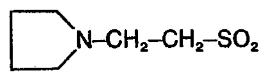 | $CH_3$ | | |
| 152 | | | 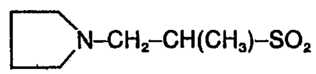 | $CH_3$ | | |
| 153 | | | 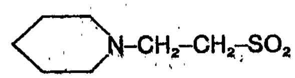 | $CH_3$ | | |
| 154 | | | 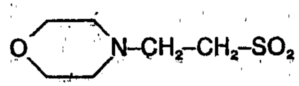 | $CH_3$ | | |
| 155 | | | 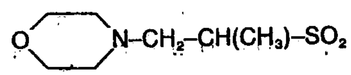 | $CH_3$ | | |
| 156 | | | 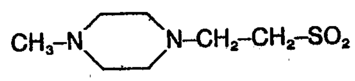 | $CH_3$ | | |
| 157 | | | 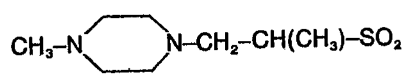 | $CH_3$ | | |
| 158 | | | $C_4H_9-NH-CH_2-CH_2-SO_2$ | $CH_3$ | | |
| 159 | | | 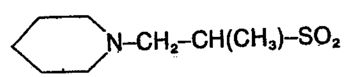 | $CH_3$ | | |

**Patentansprüche**

1. 2-Benzimidazolylcarbamidester der Formel

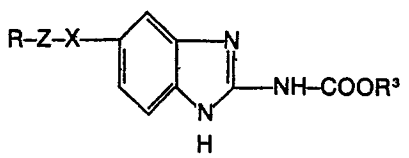 (I)

in welcher

X für ein Schwefelatom, ein Sauerstoffatom, die SO- oder $SO_2$-Gruppe steht,

Z für eine Alkylengruppe mit 1 bis 6 Kohlenstoffatomen steht,

R für die $NH_2$-, $NHR^1$- oder die $NR^1R^2$-Gruppe steht, wobei die Substituenten $R^1$ und $R^2$ gleich oder verschieden sein können und für gegebenenfalls durch Halogen, Cyan, $C_1$–$C_4$-Alkoxy oder durch die Nitrogruppe substituiertes $C_1$–$C_6$-Alkyl, gegebenenfalls durch $C_1$–$C_4$-Alkyl, $C_1$–$C_4$-Alkoxy, Halogen, Trifluormethyl, Nitro oder Cyano substituiertes Phenyl oder gegebenenfalls durch $C_1$–$C_4$-Alkyl, $C_1$–$C_4$-Alkoxy, Halogen, Trifluormethyl, Nitro, Cyano substituiertes Phenylalkyl, wobei der Alkylteil des Phenylalkylrestes 1 bis 4 Kohlenstoffatome besitzt, stehen, oder wobei $R^1$ der Gruppen $NHR^1$ oder $NR^1R^2$ mit einem Kohlenstoffatom des Substituenten Z zu einem 5- bis 7gliedrigen gegebenenfalls durch $C_1$–$C_4$-Alkyl substituierten Ring verbunden sein kann, oder wobei die Reste $R^1$ und $R^2$ zusammen mit dem Stickstoffatom einen gegebenenfalls durch $C_1-C_4$-Alkyl substituierten Azabicycloalkylrest mit 6 bis 10 Ringgliedern bilden können, oder wobei R für eine gegebenenfalls durch O, S, NH oder $NR^1$ unterbrochene und/oder durch ein oder zwei ($C_1$–$C_4$)-Alkyl-Gruppen substituierte Cycloalkylenimino-, Cycloalkenylenimino- oder Cycloalkadienyleniminogruppe mit insgesamt 4 bis 7 Ringgliedern steht, und $R^3$ für ($C_1$–$C_4$)-Alkyl oder für ($C_2$–$C_6$)-Alkenyl steht, und deren physiologisch verträglichen Salze.

2. 2-Benzimidazolylcarbamidester gemäss der Formel (I) aus Anspruch 1, dadurch gekennzeichnet, dass

X für Sauerstoff oder Schwefel steht,

Z für eine geradkettige oder verzweigte Alkylengruppe mit 1 bis 6 Kohlenstoffatomen steht,

R für die $NHR^1$ oder die $NR^1R^2$-Gruppe steht, wobei $R^1$ und $R^2$ gleich oder verschieden sein können und für ($C_1$–$C_6$)-Alkyl stehen, oder wobei $R^1$ der Gruppen $NHR^1$ oder $NR^1R^2$ mit einem Kohlenstoffatom der Alkylengruppe Z zu einem 5- oder 6gliedrigen Ring verbunden ist und $R^2$ der Gruppe $NR^1R^2$ für ($C_1$–$C_6$)-Alkyl steht, oder wobei die Substituenten $R^1$ und $R^2$ zusammen mit dem durch sie eingeschlossenen Stickstoffatom einen Azabicycloalkylrest mit 6 bis 10 Ringgliedern bilden, oder wobei R für eine gegebenenfalls durch Sauerstoff, Schwefel, eine NH- oder eine $NR^4$-Gruppe unterbrochene und gegebenenfalls durch ein oder zwei ($C_1$–$C_4$)-Alkyl-Gruppen substituierte Cycloalkyleniminogruppe mit insgesamt 5 bis 7 Ringgliedern steht, und $R^4$ ($C_1$–$C_4$)-Alkyl bedeutet, und $R^3$ für ($C_1$–$C_4$)-Alkyl steht, und deren physiologisch verträglichen Salze.

3. Verfahren zur Herstellung von 2-Benzimidazolylcarbamidestern der Formel

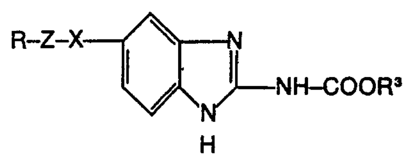 (I)

in welcher

X, Z, R und $R^3$ die im Anspruch 1 angegebene Bedeutung haben, dadurch gekennzeichnet, dass man

a) o-Phenylen-diaminderivate der Formel in der

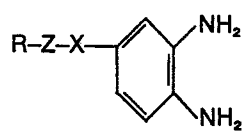 (II)

R, X und Z die oben angegebene Bedeutung besitzen, mit Säurederivaten der Formel

$$Y\text{–}COOR^3 \quad (III)$$

in welcher

$R^3$ die oben angegebene Bedeutung besitzt und

Y für einen der folgenden Reste steht:

$N{\equiv}C\text{–}NH\text{–}$, 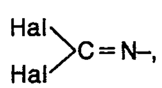 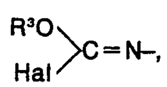 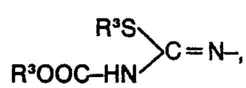

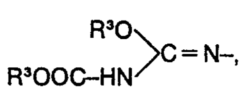 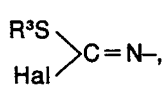 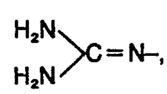 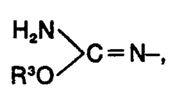

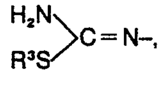 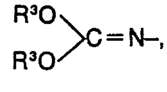 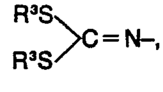 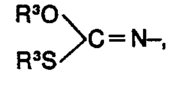

wobei $R^3$ die oben angegebene Bedeutung besitzt und Hal für Halogen steht,
umsetzt, oder dass man

b) 2-Aminobenzimidazol-Derivate der Formel

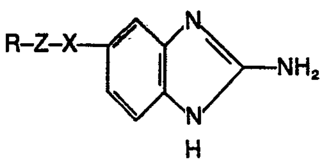
(IV)

in welcher

R, X und Z die oben angegebene Bedeutung besitzen, mit einem Kohlensäurederivat der Formel

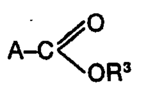
(V)

in welcher

$R^3$ die oben angegebene Bedeutung besitzt und

A für Halogen, Alkoxy oder den Rest $OCOOR^3$ steht, wobei $R^3$ wiederum die oben angegebene Bedeutung besitzt,

gegebenenfalls in Gegenwart von Säurebindemitteln umsetzt
oder dass man zum Erhalt von Verbindungen der Formel (I), bei welchen X für die SO- oder $SO_2$-Gruppe steht,

c) Arylthiobenzimidazolylcarbamidsäureester der Formel

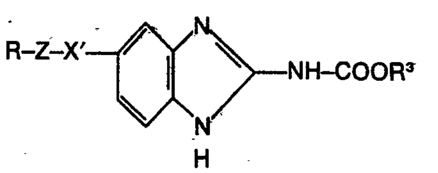
(VI)

in welcher

R und Z die oben angegebene Bedeutung besitzen und

X' für ein Schwefelatom steht,

mit einem geeigneten Oxidationsmittel umsetzt, und die nach den Verfahrensvarianten a), b) oder c) erhaltenen Endprodukte nach an sich bekannten Methoden isoliert.

4. Arzneimittel, gekennzeichnet durch einen Gehalt an mindestens einem 2-Benzimidazolylcarbamidester gemäss Anspruch 1.

5. Verfahren zur Herstellung von anthelmintischen Mitteln, dadurch gekennzeichnet, dass man 2-Benzimidazolylcarbamidester gemäss Anspruch 1 mit inerten, nichttoxischen, pharmazeutisch geeigneten Trägerstoffen vermischt.

**Claims**

1. 2-Benzimidazolylcarbamic esters of the formula

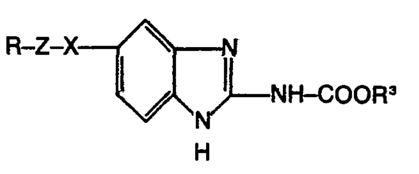
(I)

in which

X represents a sulphur atom, an oxygen atom, the SO group or the $SO_2$ group,

Z represents an alkylene group with 1 to 6 carbon atoms,

R represents the $NH_2$, $NHR^1$ or the $NR^1R^2$ group, wherein the substituents $R^1$ and $R^2$ can be identical or different and represent $C_1$–$C_6$-alkyl which is optionally substituted by halogen, cyano, $C_1$–$C_4$-alkoxy or by the nitro group, phenyl which is optionally substituted by $C_1$–$C_4$-alkyl, $C_1$–$C_4$-alkoxy, halogen, trifluoromethyl, nitro or cyano or phenylalkyl which is optionally substituted by $C_1$–$C_4$-alkyl, $C_1$–$C_4$-alkoxy, halogen, trifluoromethyl, nitro or cyano, the alkyl part of the phenylalkyl radical containing 1 to 4 carbon atoms,
or wherein

$R^1$ of the groups $NHR^1$ or $NR^1R^2$ can be linked with a carbon atom of the substituent Z to a form a 5-membered to 7-membered ring which is optionally substituted by $C_1$–$C_4$-alkyl,
or wherein

the radicals $R^1$ and $R^2$ together with the nitrogen atom can form an azabicycloalkyl radical with 6 to 10 ring members which is optionally substituted by $C_1$–$C_4$-alkyl,
or wherein

R represents a cycloalkyleneimino, cycloalkenyleneimino or cycloalkadienyleneimino group which has a total of 4 to 7 ring members and is optionally interrupted by O, S, NH or $NR^1$ and/or substituted by one or two $C_1$–$C_4$-alkyl groups, and $R^3$ represents ($C_1$–$C_4$)-alkyl or ($C_2$–$C_6$)-alkenyl, and physiologically acceptable salts thereof.

2. 2-Benzimidazolcarbamic esters according to the formula (I) from claim 1, characterised in that

X represents oxygen or sulphur,

Z represents a straight-chain or branched alkylene group with 1 to 6 carbon atoms,

R represents the $NHR^1$ or $NR^1R^2$ group,
wherein

$R^1$ and $R^2$ can be identical or different and represent ($C_1$–$C_6$)-alkyl,
or wherein

$R^1$of the groups $NHR^1$ or $NR^1R^2$ is linked with a carbon atom of the alkylene group Z to form a 5-membered or 6-membered ring and

$R^2$ of the group $NR^1R^2$ represents ($C_1$–$C_6$)-alkyl,
or wherein

the substituents $R^1$ and $R^2$ together with the nitrogen atom surrounded by them, form an azabicycloalkyl radical with 6 to 10 ring members,
or wherein

R represents a cycloalkyleneimino group which has a total of 5 to 7 ring members and is optionally interrupted by oxygen, sulphur or an NH or $NR^4$ group and optionally substituted by one or two ($C_1$–$C_4$)-alkyl groups and

$R^4$ denotes ($C_1$–$C_4$)-alkyl, and

$R^3$ represents ($C_1$–$C_4$)-alkyl, and physiologically acceptable salts thereof.

3. Process for the preparation of 2-benzimidazolylcarbamic esters of the formula

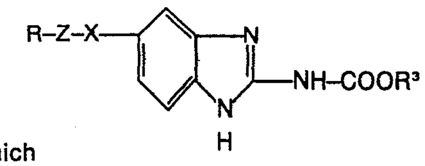

(I)

in which

X, Z, R and $R^3$ have the meaning indicated in claim 1, characterised in that

a) o-phenylene-diamine derivatives of the formula

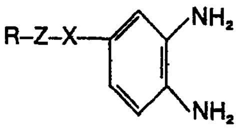

(II)

in which

R, X and Z have the meaning indicated above, are reacted with acid derivatives of the formula

$$Y{-}COOR^3 \qquad (III)$$

in which

$R^3$ has the meaning indicated above and

Y represents one of the following radicals:

$N{\equiv}C{-}NH{-}$, 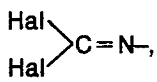 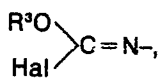 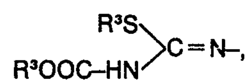

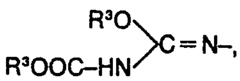 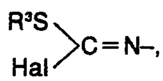 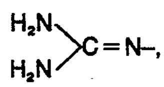 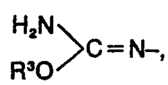

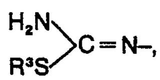 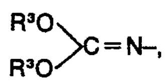 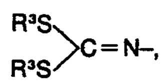 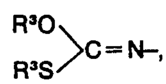

wherein

$R^3$ has the meaning indicated above and Hal represents halogen,

or in that

b) 2-aminobenzimidazole derivatives of the formula

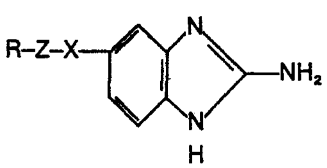

(IV)

in which

R, X and Z have the meaning indicated above, are reacted with a carbonic acid derivative of the formula

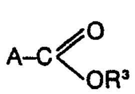

(V)

in which

$R^3$ has the meaning indicated above and

A represents halogen, alkoxy or the radical $OCOOR^3$,

wherein

$R^3$ again has the meaning indicated above, if appropriate in the presence of acid-binding agents, or in that, to obtain compounds of the formula (I) in which X represents the SO group or the $SO_2$ group,

c) arylthiobenzimidazolylcarbamic acid esters of the formula

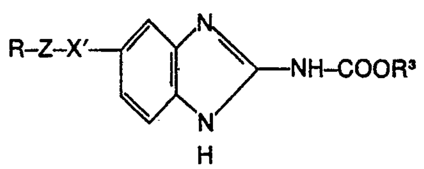

(VI)

in which

R and Z have the meaning indicated above and

X′ represents a sulphur atom,are reacted with a suitable oxidising agent, and the end products obtained by process variants a), b) or c) are isolated by methods which are in themselves known.

4. Medicaments, characterised in that they contain at least one 2-benzimidazolylcarbamic ester according to claim 1.

5. Process for the preparation of anthelmintic agents, characterised in that 2-benzimidazolylcarbamic esters according to claim 1 are mixed with inert, non-toxic, pharmaceutically suitable excipients.

**Revendications**

1. Esters 2-benzimidazolylcarbamiques de formule:

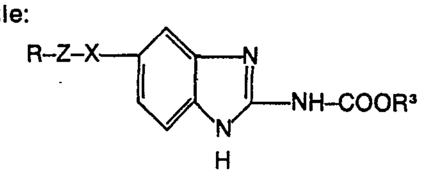

(I)

dans laquelle:

X représente un atome de soufre, un atome d'oxygène, le groupe SO ou le groupe $SO_2$,

Z représente un groupe alkylène en $C_1$–$C_6$,

R représente le groupe $NH_2$, $NHR^1$ ou $NR^1R^2$, où les substituants $R^1$ et $R^2$ peuvent être identiques ou différents et représentent chacun un halogène ou un groupe cyano, alcoxy en $C_1$–$C_4$, ou un groupe alkyle en $C_1$–$C_6$ substitué par le groupe nitro, un groupe phényle éventuellement substitué par alkyle en $C_1$–$C_4$, alcoxy en $C_1$–$C_4$, halogène, trifluorométhyle, nitro ou cyano ou un groupe phénylalkyle éventuellement substitué par alkyle en $C_1$–$C_4$, alcoxy en $C_1$–$C_4$, halogène, trifluorométhyle, nitro, cyano, dans lequel le groupe alkyle du reste phénylalkyle contient 1 à 4 atomes de carbone, ou bien le reste $R^1$ des groupes $NHR^1$ ou $NR^1R^2$ peut être lié avec un atome de carbone du substituant Z à un noyau à 5 à 7 chaînons éventuellement substitué par un alkyle en $C_1$–$C_4$, ou bien les restes $R^1$ et $R^2$ peuvent former avec l'atome d'azote un reste azabicycloalkyle à 6 à 10 chaînons éventuellement substitué par un alkyle en $C_1$–$C_4$ ou bien R représente un groupe cycloalkylèneimino, cycloalkénylèneimino ou cycloalkadiénylèneimino ayant au total 4 à 7 chaînons, éventuellement interrompu par O, S, NH ou $NR^1$ et/ou substitué par un ou deux groupes alkyle en $C_1$–$C_4$, et

$R^3$ représente un alkyle en $C_1$–$C_4$ ou un alkényle en $C_2$–$C_6$, et leurs sels physiologiquement acceptables.

2. Esters 2-benzimidazolylcarbamiques de formule (I) selon la revendication 1, caractérisés en ce que:

X représente l'oxygène ou le soufre,

Z représente un groupe alkylène à chaîne droite ou ramifiée en $C_1$–$C_6$,

R représente le groupe $NHR^1$ ou $NR^1R^2$ où $R^1$ et $R^2$ peuvent être identiques ou différents et représentent des alkyles en $C_1$–$C_6$, ou bien le reste $R^1$ des groupes $NHR^1$ ou $NR^1R^2$ est lié avec un atome de carbone du groupe alkylène Z en un cycle à 5 ou 6 chaînons et le reste $R^2$ du groupe $NR^1R^2$ représente un groupe alkyle en $C_1$–$C_6$, ou bien les substituants $R^1$ et $R^2$ forment avec l'atome d'azote inclus avec ceux-ci un reste azabicycloalkyle à 6 à 10 chaînons, ou bien R représente un groupe cycloalkylèneimino ayant au total 5 à 7 chaînons, éventuellement interrompu par l'oxygène, le soufre, un groupe NH ou un groupe $NR^4$ et éventuellement substitué par un ou deux groupes alkyle en $C_1$–$C_4$ et $R^4$ représente un alkyle en $C_1$–$C_4$, et $R^3$ représente un groupe alkyle en $C_1$–$C_4$, et leurs sels physiologiquement acceptables.

3. Procédé pour la préparation d'esters 2-benzimidazolylcarbamiques de formule:

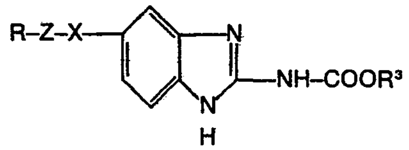 (I)

dans laquelle:

X, Z, R et $R^3$ ont la signification indiquée à la revendication 1, caractérisé en ce que

a) ou fait réagir les dérivés de o-phénylènediamine de formule:

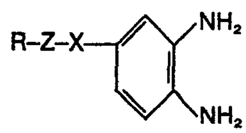 (II)

dans laquelle:

R, X et Z ont la signification indiquée ci-dessus, avec des dérivés d'acides de formule:

$$Y\text{–}COOR^3 \quad (III)$$

dans laquelle:

$R^3$ a la signification indiquée ci-dessus, et

Y représente l'un des restes suivants:

$N{\equiv}C\text{–}NH\text{–}$, 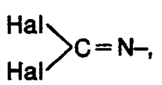 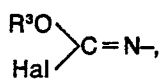 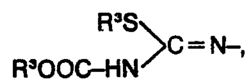

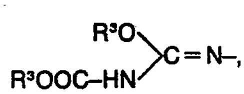 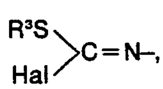 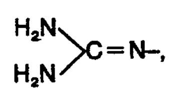 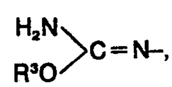

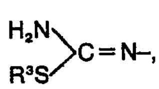 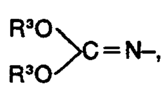 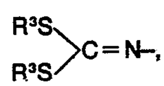 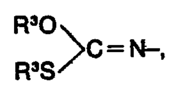

où $R^3$ a la signification indiquée ci-dessus et Hal représente un halogène,

ou bien en ce que:

b) on fait réagir des dérivés de 2-aminobenzimidazole de formule:

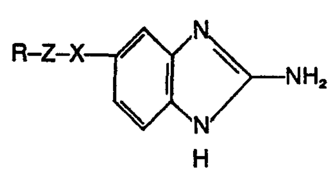 (IV)

dans laquelle:

R, X et Z ont la signification indiquée ci-dessus avec un dérivé d'acide carbonique de formule:

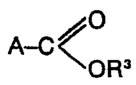

(V)

dans laquelle:

$R^3$ a la signification indiquée ci-dessus, et

A représente un halogène, un alcoxy ou le reste $OCOOR^3$ où $R^3$ a de nouveau la signification indiquée ci-dessus, éventuellement en présence d'accepteurs d'acides, ou en ce que, pour obtenir des composés de formule (I), dans lesquels X représente le groupe SO ou $SO_2$,

c) on fait réagir des esters d'acides arylthiobenzimidazolylcarbamiques de formule:

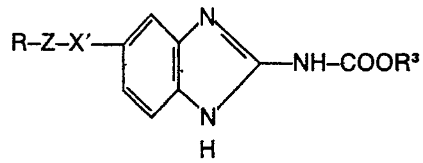

(VI)

dans laquelle:

R et Z ont la signification indiquée ci-dessus et

X' représente un atome de soufre,

avec un agent oxydant approprié, et on isole par des méthodes connues en soi les produits finals obtenus selon les variantes a), b) ou c) du procédé.

4. Médicaments, caractérisés en ce qu'ils contiennent au moins un ester 2-benzimidazolylcarbamique selon la revendication 1.

5. Procédé pour la préparation d'agents anthelmintiques, caractérisé en ce que l'on mélange des esters 2-benzimidazolylcarbamiques selon la revendication 1 avec des supports inertes non toxiques appropriés en pharmacie.